**Europäisches Patentamt**

**European Patent Office**

**Office européen des brevets**

(19)

(11) Veröffentlichungsnummer: **0 337 513 B1**

(12) # EUROPÄISCHE PATENTSCHRIFT

(45) Veröffentlichungstag der Patentschrift:
**11.12.91 Patentblatt 91/50**

(51) Int. Cl.⁵: **A61F 2/34**

(21) Anmeldenummer: **89111097.5**

(22) Anmeldetag: **09.03.85**

(54) **Prothetische Hüftpfanne.**

(30) Priorität: **14.04.84 DE 8411765 U**

(43) Veröffentlichungstag der Anmeldung:
**18.10.89 Patentblatt 89/42**

(60) Veröffentlichungsnummer der früheren
Anmeldung nach Art. 76 EPü: **0 159 510**

(45) Bekanntmachung des Hinweises auf die
Patenterteilung:
**11.12.91 Patentblatt 91/50**

(84) Benannte Vertragsstaaten:
**CH DE FR GB IT LI**

(56) Entgegenhaltungen:
**DE-A- 2 925 089**
**DE-A- 2 932 744**
**DE-B- 2 306 552**
**DE-U- 8 212 689**
**FR-A- 2 194 123**
**GB-A- 2 069 338**

(73) Patentinhaber: **HOWMEDICA**
**INTERNATIONAL, INC. Zweigniederlassung**
**Kiel**
**Professor-Küntscher-Strasse 1-5**
**W-2314 Schönkirchen üb. Kiel (DE)**

(72) Erfinder: **Beck, Heinrich, Prof. Dr.**
**Rudelsweier 12 1/2**
**W-8520 Erlangen (DE)**
Erfinder: **Richter, Karl Manfred, Dipl.-Ing.**
**Haferkamp 14**
**W-2304 Wendtorf (DE)**

(74) Vertreter: **Dipl.-Ing. H. Hauck Dipl.-Ing. E.**
**Graalfs Dipl.-Ing. W. Wehnert Dr.-Ing. W.**
**Döring**
**Neuer Wall 41**
**W-2000 Hamburg 36 (DE)**

## Beschreibung

Die Erfindung bezieht sich auf eine prothetische Hüftpfanne nach dem Oberbegriff des Patentanspruchs 1.

Aus der FR-A-21 94 123 ist eine prothetische Hüftpfanne bekannt geworden, die aus einer metallischen äußeren Schale und einer aus Kunststoff bestehenden inneren Schale zur Aufnahme des Gelenkkopfes zusammengesetzt ist, wobei die äußere Schale einen annähernd zylindrischen Abschnitt aufweist, an dessen offenem Ende ein radial umlaufender Kragen angeformt ist. Die Außenseite der äußeren Schale ist ebenfalls sowohl im Bodenbereich als auch im Wandbereich aufgerauht. Im Wand bereich ist eine pyramidenartige Aufrauhung vorgesehen.

Ausgehend von diesem Stand der Technik ist es die Aufgabe der Erfindung, eine Pfanne zu schaffen, die ohne Verwendung von Knochenzement einen wirksamen Sitz in der vorbereiteten Bohrung in der Hüfte zu gewährleistet.

Gelöst wird diese Aufgabe mit einer Pfanne, an deren äußerer Schale am zylindrischen Umfang axiale Nuten geformt sind, die vorzugsweise Zahnprofil haben. Die axialen Nuten dienen dabei zur Führung und zur Rotationssicherung. Umfangsnuten, die vorzugsweise am distalen Ende der Pfanne vorgesehen sind, sollen eine Extraktion verhindern.

Um auch dem Boden der äußeren Schale der Hüftpfanne eine Oberflächenvergrößerung zu verleihen, sieht eine Ausgestaltung der Erfindung vor, daß der Boden der äußeren Schale an der Außenseite eine Reihe von Erhebungen aufweist, die vorzugsweise pyramidenförmig sind.

Die äußere Schale der prothetischen Pfanne wird nach einer Ausgestaltung der Erfindung aus einer Eisen und Aluminium enthaltenden Titanlegierung geformt. Die Legierung lautet vorzugsweise $TiAl_5Fe_{2,5}$. Eine derartige Legierung ist außer ordentlich gewebeverträglich und besitzt zudem einen Elastizitätsmodul, der erheblich kleiner ist als bei den herkömmlichen Legierungen. Im Fall der bevorzugten Ausführungsform ist der E-Modul lediglich halb so groß. Dadurch nähert sich die Elastizität der prothetischen Hüftpfanne der des Knochens an, so daß das Ausmaß von Relativbewegungen zwischen Knochen und Prothese bei einer dynamischen Belastung reduziert wird. Dadurch ist auch die Belastung der Prothese im Hinblick auf ihre Lockerung der Verankerung im Knochen verringert.

Der innere Teil der prothetischen Hüftpfanne besteht in der Regel aus Kunststoffmaterial, vorzugsweise Polyethylen. Damit die Gelenkkugel beim Einsetzen in die innere Schale nicht gegen die metallische äußere Schale schlägt und dadurch beschädigt wird, sieht eine weitere Ausgestaltung der Erfindung vor, daß die Innenschale einen sich gegen die Vorderseite des Kragens der äußeren Schale legenden radialen Flansch aufweist. Dieser kann sehr dünn gehalten werden, sorgt jedoch dafür, daß bei nicht zentriertem Einführen der Gelenkkugel diese allenfalls gegen den Kunststoffflansch stößt, dabei jedoch nicht beschädigt wird.

Die Erfindung wird nachfolgend anhand von Zeichnungen näher erläutert.

Fig. 1 zeigt halb im Schnitt, halb in Seitenansicht den äußeren Teil der erfindungsgemäßen Hüftkappe.

Fig. 2 zeigt eine Oberansicht des Teils nach Fig. 1.

Fig. 3 zeigt den Ausschnitt 3 der Darstellung nach Fig. 1 in vergrößerter Form.

Fig. 4 zeigt teils im Schnitt, teils in Seitenansicht den inneren Teil der Hüftkappe.

Bevor auf die in den Zeichnungen dargestellten Einzelheiten näher eingegangen wird, sei vorangestellt, daß jedes der beschriebenen Teile für sich oder in Verbindung mit Merkmalen der Ansprüche von Bedeutung ist.

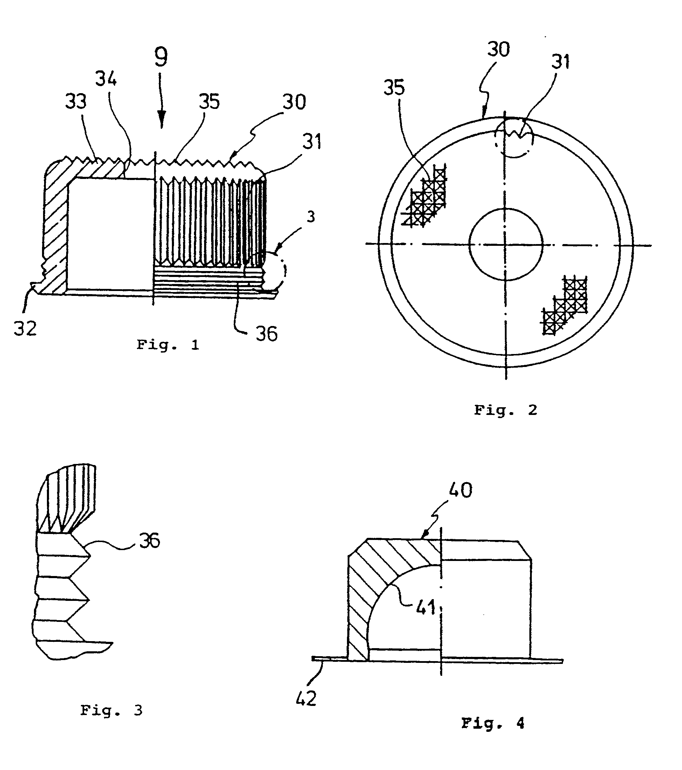

Die Außenschale der Hüftpfanne gemäß den Figuren 1 bis 3 ist allgemein mit 30 bezeichnet. Sie besteht vorzugsweise aus dem gleichen Material wie ein in Verbindung damit implantierter Schaft. Im zylindrischen Mantel ist eine Reihe von achsparallelen Nuten 31 geformt, die im Querschnitt dreieckförmig sind und so aneinanderliegen, daß dazwischen dreieckförmige Zähne gebildet sind. Der Mantel ist an der offenen Seite mit einem radial nach außen weisenden Flansch 32 versehen. Auf der gegenüberliegenden Seite ist ein Boden 33 geformt mit einer mittigen Öffnung 34. Auf der Außenseite des Bodens 33 ist ein Muster von pyramidenartigen Erhebungen 35 geformt.

Die Nuten 31 enden vor dem Eintrittsende der äußeren Schale 30. Dazwischen sind drei Umfangsnuten 36 geformt, die zwischen sich dreieckförmige Rippen oder Zähne bilden. Die Tiefe der Nuten 36 entspricht der Tiefe der Nuten 31, wie aus Fig. 3 zu erkennen. Die den Längsnuten 31 benachbarte Umfangsnut 36 ist so gelegt, daß die eine Nutflanke von den Enden der Zähne zwischen den Längsnuten 31 gebildet wird (siehe ebenfalls Fig. 3). Die äußere Schale 30 wird ohne Knochenzement in eine vorgebohrte Öffnung des Beckenknochens eingesetzt. Die Längsnuten 31 verhindern die Rotation, und die Umfangsnuten 36 wirken der Extraktion entgegen.

Der zylindrische Innenraum der äußeren Schale 30 nimmt eine Innenschale 40 aus Polyethylen auf. Die Innenschale 40 ist mit den Außenabmessungen so gewählt, daß sie passend in den Innenraum der äußeren Schale 30 einsetzbar sind. Die Innenschale 40 weist eine kugelige Höhlung 41 auf zur passenden und schnappenden Aufnahme der nicht gezeigten Gelenkkugel. Die Innenschale 40 besitzt ebenfalls an der offenen Seite einen radial nach außen weisenden Flansch 42, der sich von unten gegen die Stirnseite und den Flansch 32 der äußeren Schale 30 anlegt. Der Flansch 42, der verhältnismäßig dünn ausgeführt ist, verhindert, daß die Gelenkkugel beim Einsetzen gegen metallisches Material stößt.

## Patentansprüche

1. Prothetische Hüftgelenkpfanne mit einer metallischen äußeren Schale (30) und einer vorzugsweise aus Kunststoff bestehenden inneren Schale (40) zur Aufnahme des Gelenkkopfes, wobei die äußere Schale (30) einen annähernd zylindrischen Abschnitt aufweist, an dessen offenem Ende ein radial umlaufender Kragen (32) angeformt ist, dadurch gekennzeichnet, daß am zylindrischen Umfang der äußeren Schale (30) axiale Nuten (31) geformt sind, die vorzugsweise ein Zahnprofil haben.

2. Hüftgelenkpfanne nach Anspruch 1, dadurch gekennzeichnet, daß am zylindrischen Umfang der äußeren Schale (30) parallele Umfangsnuten (36) geformt sind, die vorzugsweise ein Zahnprofil bilden und nahe dem Kragen (32) zwischen diesem und den axialen Nuten (31) angeordnet sind, und daß beide Nuten (31, 36) annähernd gleich tief ausgebildet sind.

3. Hüftgelenkpfanne nach Anspruch 1 oder 2, dadurch gekennzeichnet, daß der Boden (33) der äußeren Schale (30) an der Außenseite eine Reihe von Erhebungen (35) aufweist.

4. Hüftgelenkpfanne nach Anspruch 3, dadurch gekennzeichnet, daß die Erhebungen (35) pyramidenförmig sind.

5. Hüftgelenkpfanne nach einem der Ansprüche 1 bis 4, dadurch gekennzeichnet, daß die äußere Schale aus einer Eisen und Aluminium enthaltenden Metallegierung gußgeformt ist.

6. Hüftgelenkpfanne nach Anspruch 5, dadurch gekennzeichnet, daß die Metallegierung $TiAL_5Fe_{2,5}$ ist.

7. Hüftgelenkpfanne nach einem der Ansprüche 1 bis 4, dadurch gekennzeichnet, daß die Innenschale (40) einen sich gegen die Vorderseite des Kragens (32) der äußeren Schale (30) liegenden radialen Flansch (42) aufweist.

## Claims

1. Prosthetic acetabulum comprising a metallic outer cup (30) and an inner cup (40) preferably consisting of synthetic material for the accommodation of the joint head, the outer cup (30) comprising an approximately cylindrical portion with a collar (32) radially and circumferentially extending at the open end thereof, characterized in that axial grooves (31) are formed at the cylindrical circumference of the outer cup (30), preferably having a tooth profile.

2. Acetabulum according to claim 1, characterized in that parallel grooves (36) are formed at the cylindrical circumference of the outer cup (30) which preferably form a tooth profile and are arranged near the collar (32) between this one and the axial grooves (31), and in that the bottom of both grooves (31, 36) approximately extend at the same level.

3. Acetabulum according to claim 1 or 2, characterized in that the bottom (33) of the outer cup (30) comprises a series of elevations (35) at the outer surface thereof.

4. Acetabulum according to claim 3, characterized in that the elevations (35) are pyramidally formed.

5. Acetabulum according to one of the claims 1 to 4, characterized in that the outer cup is formed by casting of a metal alloy containing iron and aluminum.

6. Acetabulum according to claim 5, characterized in that the metal alloy is $TiAl_5Fe_{2,5}$.

7. Acetabulum according to one of the claims 1 to 4, charecterized in that the inner cup (40) has a radial flange (42) which comes to lie against the front surface of the collar (32) of the outer cup (30).

## Revendications

1. Cupule pour prothèse de l'articulation de la hanche, comprenant une coquille extérieure (30) métallique, et une coupelle intérieure (40), de préférence en matière synthétique, destinée à recevoir la tête de l'articulation, la coquille extérieure (30) ayant une section à peu près cylindrique sur l'extrémité ouverte de laquelle est formée une collerette périphérique radiale (32), caractérisée en ce que des rainures axiales (31) sont formées sur le pourtour cylindrique de la coquille extérieure (30), ces rainures ayant de préférence un profil dentelé.

2. Cupule selon la revendication 1, caractérisée en ce que des rainures parallèles (36) sont formées sur la périphérie cylindrique de la coquille extérieure (30), ont de préférence un profil dentelé et sont placées près de la collerette (32) et entre celle-ci et les rainures axiales, les deux types de rainures (31, 36) ayant à peu près la même profondeur.

3. Cupule selon la revendication 1 ou 2, caractérisée en ce que le fond (33) de la coquille extérieure (30) présente, sur sa face extérieure, une série de saillies (35).

4. Cupule selon la revendication 3, caractérisée en ce que les saillies (35) sont en forme de pyramide.

5. Cupule selon l'une des revendications 1 à 4, caractérisée en ce que la coquille extérieure est en un alliage métallique contenant du fer et de l'aluminium.

6. Cupule selon la revendication 5, caractérisée en ce que l'alliage métallique est $Ti\text{-}Al_5\text{-}Fe_{2,5}$.

7. Cupule selon l'une des revendication 1 à 4, caractérisée en ce que la coquille intérieure (40) comporte un flasque radial (42) qui vient en appui contre la face avant de la collerette (32) de la coquille extérieure (30).

Fig. 1

Fig. 2

Fig. 3

Fig. 4